# FASCICULE DE BREVET EUROPEEN

(11) **EP 2 644 173 B1**
(45) Date de publication et mention de la délivrance du brevet: **27.04.2016**
(21) Numéro de dépôt: 13171315.8
(22) Date de dépôt: 29.04.2005
(51) Int. Cl.: A61F 9/00, A61N 1/30

(54) **Dispostif d'iontophorèse oculaire diminuant les irritations**
Irritationsvermindernde Iontophoresevorrichtung für das Auge
Irritation-reducing ocular iontophoretic device

(30) Priorité: 30.04.2004 FR 0404673; 03.06.2004 US 861117
(43) Date de publication de la demande: 02.10.2013
(62) Demande divisionnaire de: 05763701.9
(73) Titulaire: Eyegate Pharma SAS, 75012 Paris (FR)
(72) Inventeur: Roy, Pierre, F-75019 Paris (FR)
(74) Mandataire: Murphy, Colm Damien

(56) Documents cités:
- WO-A2-03/030989
- US-A- 4 474 570
- US-A- 6 154 671
- US-A1- 2002 035 345
- US-B1- 6 327 496

## Description

### DOMAINE TECHNIQUE

La présente invention concerne un dispositif de iontophorèse oculaire pour délivrer des principes actifs (dans l'oeil).

Le principe de la iontophorèse oculaire est l'application d'un champ électrique à une substance électrolytique contenant les principes actifs, pour transporter ces principes actifs dans le corps ou l'organe à traiter, à travers les membranes biologiques de l'oeil.

L'oeil, comme le cerveau, est protégé du système veineux central par des barrières biologiques (hémato-oculaires, hémato-acqueuse hémato-rétinienne) rendant très difficile l'administration de médicaments notamment dans le segment postérieur de l'oeil, en particulier dans la rétine, avec une concentration suffisante.

### ARRIERE-PLAN TECHNOLOGIQUE

La voie systémique (orale ou intraveineuse) ne permet ainsi d'administrer qu'une très faible fraction (quelques %) de la quantité initiale dans les tissus internes des yeux, et devient donc vite insuffisante.

C'est pourquoi des techniques d'administration locales de médicaments dans l'oeil ont été ou sont en développement, parmi lesquelles on compte :
- Les injections directes autour de l'oeil (péribulbaire, rétrobulbaire) ou dans l'oeil (intraoculaires), qui restent très traumatisantes. En outre, la drogue est soit rapidement diluée, disparaissant alors du vitré en quelques jours, soit repassée en systémique. Ce mode d'administration présente aussi des risques d'infection, de saignements, de cataracte et de détachement de rétine. Enfin, des affections comme le glaucome ne peuvent pas être traités de cette façon en raison de l'augmentation de la pression intraoculaire.
- Les applications topiques par gouttes, qui ne traitent pas le segment postérieur car la pénétration est très limitée (typiquement moins de 5%) et qui ne permettent pas d'atteindre des concentrations thérapeutiques au-delà du segment antérieur. De plus, les larmes lavant la drogue rapidement, les fluides oculaires s'opposent à la pénétration et le chemin de diffusion est alors assez long. Les applications doivent être répétées fréquemment.
- La thérapie photodynamique consiste à injecter de façon systémique un médicament et à l'activer de façon locale en utilisant un laser d'une certaine longueur d'onde, profitant alors de la transparence de la cornée. Cependant, des inconvénients subsistent : le patient doit rester dans l'obscurité, le médicament doit être modifié par l'ajout d'un agent photosensible qui bloque son activité jusqu'à l'activation, et le médecin doit disposer d'un matériel relativement onéreux.
- Les inserts sont des réservoirs de médicament placés à la surface de l'oeil (de façon non invasive), comme des lentilles ou de préférence dans le sac conjonctival, et qui permettent de délivrer les médicaments de façon continue ou programmée. De nombreux systèmes ont été développés, soit sous la forme d'une lentille ou d'un anneau, soit d'un petit réservoir de forme lenticulaire ou tubulaire déposé dans le sac conjonctival. Les inconvénients principaux de ces systèmes sont d'une part un passage limité dans le segment postérieur qui limite leur utilisation aux pathologies du segment antérieur (inflammations, conjonctivites) et d'autre part, le risque d'expulsion de l'insert.
- Les implants intraoculaires de libération programmée de médicaments sont mis en place chirurgicalement dans le vitré comme le Vitrasert^{®} de Baush & Lomb, éventuellement fixés sur la sclère comme l'implant hélicoïdal enduit d'une couche de polymère re-larguant le médicament (InnoRx/Surmodics) ou bien sont bio-érodables/biodégradables (Surodex^{®} d'Oculex, maintenant Allergan). Un des inconvénients de ce type de dispositifs est de se mouvoir librement dans le vitré et de risquer de toucher la rétine en augmentant la concentration locale de médicament jusqu'à un niveau toxique. Il est possible de suturer l'implant mais cela requiert une incision relativement large (5mm). Un autre inconvénient est de devoir être remplacé régulièrement. Enfin, il n'est pas possible d'interrompre ou d'accélérer le traitement en fonction de l'évolution de la pathologie à traiter.

La iontophorèse oculaire est aussi une technique d'administration locale de médicaments dans l'oeil, qui permet de pallier la plupart des inconvénients des autres techniques précitées. Elle permet en outre d'obtenir des concentrations et des temps de résidence intraoculaire de façon non invasive égaux ou supérieurs aux techniques précédentes.

Les dispositifs de iontophorèse sont typiquement constitués d'une source de champ électrique constant couplée à 2 électrodes, nommées électrodes active et passive. L'électrode active agit sur l'électrolyte contenant le(s) principe(s) actif(s), l'électrode passive sert d'électrode de retour et permet de boucler le circuit électrique à travers le corps du patient.

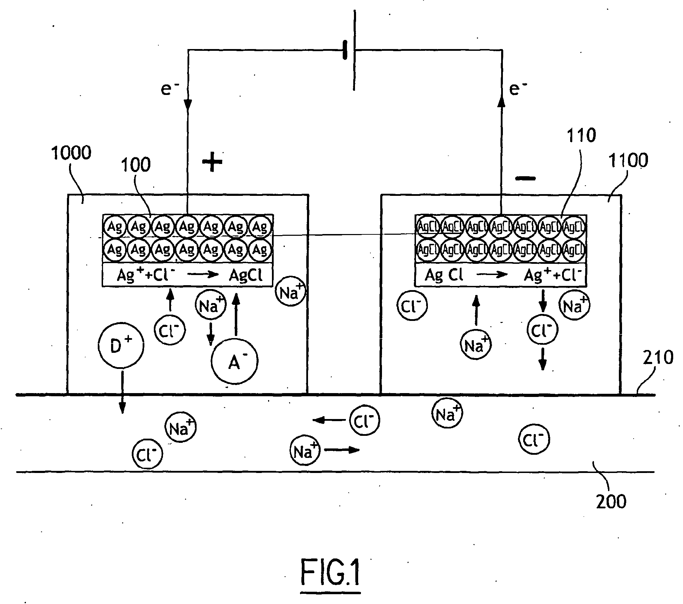

A un système d'électrodes métalliques à réactions d'oxydo-réduction, dans lequel des particules métalliques toxiques sont susceptibles de passer en solution, ont été préférées un système d'électrodes Ag/AgCl pour mettre en oeuvre la iontophorèse dont le principe est illustré en référence à la figure 1. Une telle iontophorèse est mise en oeuvre dans un tissu vivant 200 à travers sa surface 210, cette surface vivante 210 étant en contact d'une part avec la solution saline d'un premier compartiment (ici anodique) 1000 contenant l'électrode active 100 (ici l'anode) en Ag et d'autre part avec la solution saline d'un deuxième compartiment (ici cathodique) 1100 contenant l'électrode passive 110 (ici la cathode) en AgCl. Le compartiment anodique 1000 contient en outre un principe actif D sous une forme ionisable (donnant alors un contre-ion A⁻ lorsque ionisé). Les polarisations des électrodes 100 et 110 créent alors une électromigration des espèces ioniques en solution, y compris du principe actif ionisé qui franchit alors la surface vivante 210, et est ainsi introduit dans le tissu vivant 200. Cette iontophorèse implique alors que de l'AgCl se dépose sur l'électrode active 100, et que de l'AgCl se décompose sur l'électrode passive 110 pour fournir en solution des ions chlorures. Ce principe nécessite l'utilisation d'un médicament en solution dans l'eau salée, les ions Na⁺ et Cl⁻ rentrant alors en compétition avec le principe actif, diminuant ainsi sensiblement le rendement de la iontophorèse.

Bien que la iontophorèse ait été utilisée cliniquement pour délivrer des médicaments en dermatologie depuis plusieurs décades, c'est une technique encore relativement récente en ophtalmologie.

Et l'oeil étant un organe extrêmement différent et particulièrement plus fragile sur beaucoup d'aspects (tels que la transparence de la cornée, la sensibilité de la muqueuse,...) que la plupart des autres parties du corps animal, la iontophorèse oculaire présente des spécificités techniques très différentes de celles des autres types d'iontophorèse (intensité, densité, durée et contrôle du champ électrique à appliquer, courants de fuite dus à la présence du liquide lacrymal (dits « effet d'arc »), vigilance particulière quant aux dosages des espèces chimiques à administrer et de la présence de contaminants en solution, attention particulière aux phénomènes électrochimiques se déroulant en solution lors de l'électrolyse, etc.).

Il reste donc un besoin d'amélioration de l'efficacité de la délivrance, afin de réduire des risques de brûlures et de toxicité tissulaire, et d'irritation des tissus en général (érythème) qui peuvent limiter l'utilisation de la iontophorèse oculaire.

Une amélioration de la iontophorèse oculaire pourrait être trouvée en :
- appliquant le champ électrique préférentiellement sur la sclère plutôt que sur la cornée, afin notamment de protéger cette dernière surface très fragile et participant directement au fonctionnement de la vision ;
- minimisant la densité du champ électrique (rapport intensité/surface), donc en maximisant la surface d'application et la surface de l'électrode ;
- contrôlant plus précisément l'intensité du champ électrique appliqué, ce qui permettrait une bonne reproductibilité ;
- limitant le temps d'application pour permettre une bonne circulation du fluide lacrymal ;
- en facilitant le passage des ions dans l'oeil.

Le document FR 2 773 320 divulgue un dispositif de iontophorèse de délivrance de principes actifs autour de la cornée, comprenant un réservoir de principes actifs dans lequel est disposé une électrode active surfacique en matériau conducteur, qui procure précisément les avantages précités. Ce dispositif permet ainsi, du fait principalement d'avoir une électrode active surfacique et située à une distance déterminée de l'oeil, une répartition du champ électrique de façon assez homogène et constante sur la surface de son application.

Cependant, en utilisant ce dernier dispositif avancé, a été constaté lors d'essais cliniques que les irritations oculaires avaient certes diminuées, mais restaient présentes. Des résultats de telles expériences sont décrits dans « Iontophoresis : from the lab to the bed side » de Halhal et coll. (« Experimental Eye Research » 78 (2004) 751-757).

En outre, il serait souhaitable de diminuer encore le temps de mise en oeuvre de la iontophorèse, afin de diminuer les risques potentiels lors de la mise en oeuvre et d'améliorer le confort du patient.

Un objectif de l'invention est de diminuer les risques d'irritations oculaires par rapport à l'état de la technique.

Un autre objectif de l'invention est de prévoir un dispositif de iontophorèse oculaire qui puisse fonctionner avec des solutions ne contenant pas ou peu d'ions concurrents au principe actif ionisé, telles que des solutions salines, atteignant ainsi, pour un champ électrique donné, un rendement de délivrance du principe actif plus grand.

Un autre objectif est de réussir à atteindre le précédent objectif tout en ne diminuant pas sensiblement l'intensité et l'homogénéité du champ électrique appliqué à la solution électrolytique, permettant ainsi d'administrer de façon homogène le principe actif, et de réduire sensiblement le temps de mise en oeuvre de la iontophorèse (et donc de diminuer les risques y afférant).

Par ailleurs, un autre objectif est de proposer un dispositif de iontophorèse oculaire qui résiste aux pressions exercées par les paupières qui se ferment.

Un autre objectif est de proposer un dispositif de iontophorèse oculaire contenant un principe actif présenté sous une forme plus facilement ionisable.

### RESUME DE L'INVENTION

Le dispositif selon l'invention montre les caractéristiques de la revendication 1.

Ainsi, selon un premier aspect, il est proposé un dispositif de iontophorèse oculaire, pour la délivrance de principes actifs, comportant un réservoir de principes actifs apte à venir se positionner sur l'oeil, des principes actifs en solution dans le réservoir, une électrode active disposée dans le réservoir, et une électrode passive, qui comprend des principes actifs dissous dans de l'eau non saline. la solution qu'il contient ayant un pH compris entre 6,5 et 8,5, les principes actifs ayant un pKa compris entre environ 5,5 et 9,5 et étant liés à un adjuvant, tel que des dendrimères, des polymères, des nanoparticules, des microsphères, des liposomes ou des émulsions et ayant une forme ionique de valence supérieure ou égale à 1

On peut également proposer un procédé de traitement par iontophorèse oculaire selon lequel, pour la délivrance de principes actifs, on positionne sur l'oeil un dispositif d'iontophorèse comportant un réservoir de principes actifs, en solution, une électrode active disposée dans le réservoir, et une électrode passive, et en ce qu'on met en oeuvre une iontophorèse oculaire au moyen de ce dispositif, les principes actifs étant dissous dans de l'eau non saline, la solution qu'il contient ayant un pH compris entre 6,5 et 8,5, les principes actifs ayant un pKa compris entre environ 5,5 et 9,5 et étant liés à un adjuvant, tel que des dendrimères, des polymères, des nanoparticules, des microsphères, des liposomes ou des émulsions et ayant une forme ionique de valence supérieure ou égale à 1.

Par ailleurs, selon un autre aspect indépendant et le cas échéant complémentaire, on peut proposer un dispositif de iontophorèse oculaire, caractérisé en ce que l'électrode active comprend une couche électriquement conductrice agencée de sorte à recevoir un champ électrique apte à la polariser suffisamment pour électrolyser les principes actifs en solution, et comprend en outre une couche de protection située entre la couche conductrice et la solution, cette couche de protection réagissant sensiblement moins que la couche conductrice lors d'une électrolyse dans la solution considérée.

### BREVE DESCRIPTION DES FIGURES

D'autres caractéristiques, buts et avantages de l'invention apparaîtront mieux à la lecture de la description en illustration des figures suivantes :
La figure 1 représente un schéma de principe d'une iontophorèse selon l'état de la technique.
La figure 2 représente schématiquement un exemple de système de iontophorèse oculaire.
La figure 3 représente une électrode active d'un dispositif de iontophorèse oculaire
Les figures 4a à 4f représentent différentes formes d'électrodes actives d'un dispositif de iontophorèse oculaire.
La figure 5 représente un dispositif de iontophorèse oculaire.
Les figures 6a à 6d représentent différentes formes de la partie souple du réservoir d'un dispositif de iontophorèse oculaire.
La figure 7a et 7b représentent différentes formes de la partie rigide ou renforcée du réservoir d'un dispositif de iontophorèse oculaire selon l'invention.
Les figures 8a à 8f représentent différentes molécules liant le principe actif à administrer à différents adjuvants.

### DESCRIPTION DETAILLEE DE L'INVENTION

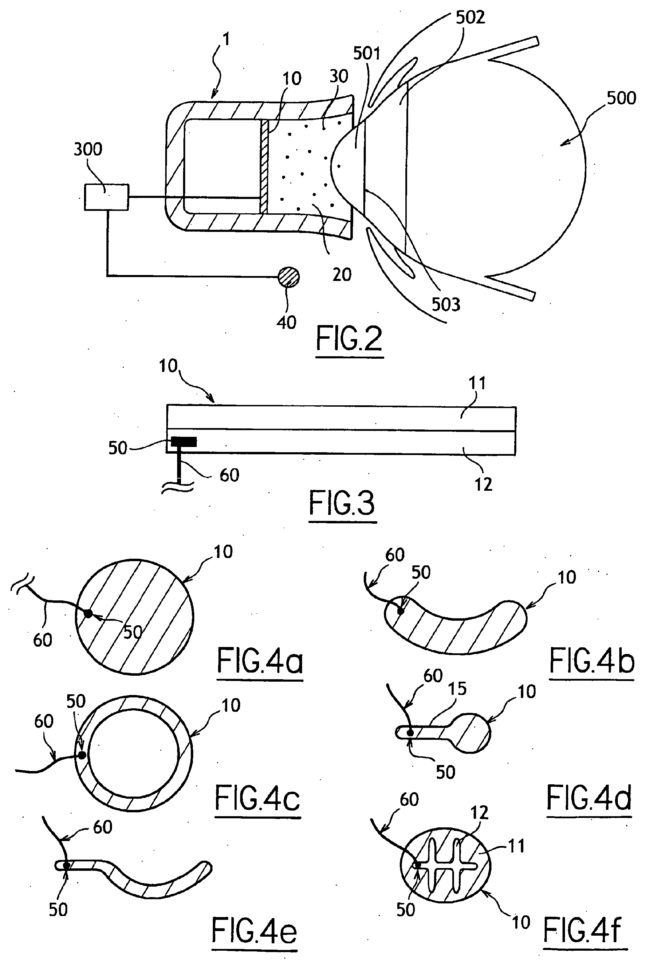

En référence à la figure 2 est représenté de façon très schématique un système de iontophorèse oculaire, comprenant un dispositif de iontophorèse 1 incluant une électrode active 10, un réservoir 20 et des principes actifs 30 stockés dans le réservoir 20, une électrode passive 40 permettant de boucler le circuit électrique, et une alimentation électrique 300 fournissant un courant continu aux électrodes 10 et 40.

L'électrode active 10 est disposée dans le réservoir 20 en l'y rapportant ou en l'y formant directement (par exemple par électrodéposition).

Le réservoir 20 est réalisé en un matériau isolant électriquement, tel qu'un plastique, un silicone, un polymère ou tout autre matériau de ce type.

Les principes actifs 30 sont disposés dans un gel ou en solution, et sont ionisables par elles-mêmes ou sous une forme qui facilite leurs ionisations (adjuvants adaptés).

L'électrode passive 40 peut être placée sur une partie du corps (afin de « boucler » le courant à travers le corps), telle que par exemple une oreille, le front, une joue.

Le dispositif 1 est placé sur le globe oculaire 500, avec ou sans insertion sous les paupières.

On pourra adapter les dimensions du dispositif 1 à la taille d'un globe oculaire 500 sachant que la taille d'un oeil humain augmente rapidement pendant les trois premières années de la vie et son diamètre passe de 16 à 23 mm (antéropostérieur), et qu'un oeil humain adulte a un volume de 5,5ml et un diamètre antéropostérieur de 24mm.

Cependant, étant donné que les dimensions oculaires sont remarquablement stables dans la population humaine et indépendantes des variations des dimensions corporelles, de la race et atteint sa maturité assez rapidement, on pourra choisir des dimensions universelles pour le dispositif 1.

Le dispositif 1 peut être adapté pour administrer les principes actifs 30 à travers :
- uniquement la cornée 501, ou
- la sclère 502 et la cornée 501, ou
- uniquement la sclère 502.

La cornée 501 représente environ 5% de la surface oculaire totale (cornée 1cm² et sclère 17 cm²+/-1,5 cm²) et rejoint la sclère 502 au niveau du limbe 503. Le diamètre du limbe 503 chez l'homme est de 11,7mm.

La cornée 501 est un tissu transparent, avasculaire, son rayon de courbure chez l'homme est de 7,8mm, son épaisseur de 0,7mm au centre et 0,5mm à la périphérie et se situe en face de la rétine fonctionnelle. Elle est composée de 3 couches (l'épithélium, la stroma, l'endothélium) dont une (l'épithélium) est généralement le facteur limitant, puisqu'elle constitue une barrière favorisant les substances lipophiles et excluant presque totalement les macromolécules d'une taille supérieure à 10 Å.

La sclère 502 est un tissu élastique et microporeux, contenant 70% d'eau, elle couvre pratiquement le reste de la surface oculaire et a une épaisseur moyenne de 0,53mm+/-0,14mm au limbe cornéo-scléral 503. Son épaisseur diminue ensuite à 0,3mm+/-0,17mm à l'équateur et augmente ensuite progressivement à 0,9 à 1,0mm vers le nerf optique. Elle comprend la conjonctive qui est une muqueuse fine et vascularisée. La limite d'exclusion de la taille des particules est comprise entre 20 000 Da et 150 000 Da.

Pour la sclère 502, le facteur limitant le passage des molécules est plus la taille de ces molécules que la lipophilicité.

La sclère 502 est plus perméable que la cornée.

Le consensus actuel sur la tolérance respective de la sclère 502 et de la cornée 501 à la densité de courant est de 100 et 20 mA/cm² respectivement.

Ci-après sont présentés la comparaison des grandeurs physiques et biologiques associées à la cornée 501 et à la sclère 502 :

| | **Cornée** | **Sclère** |
|---|---|---|
| **Epaisseur mini (mm)** | 0,5 | 0,39 |

| | | |
|---|---|---|
| **Épaisseur maxi (mm)** | 0,7 | 1,0 |
| **Surface (cm²)** | 1 | 16,3 |
| **Taille maxi des molécules (Da & nm)** | 1 000Da | 150 000Da |
| **Densité de courant maxi (mA/cm²)** | 20 | 100 |

Selon une variante préférée de l'invention, on pourra choisir de dispenser les principes actifs 30 à travers la sclère 502, étant donné qu'elle présente des caractéristiques favorisant la iontophorèse (plus grande perméabilité, plus grande surface d'administration, plus favorable à l'application de forts courants) et que la cornée 501 est une partie de l'oeil bien plus critique que la sclère 502.

Selon cette dernière variante, on prévoira alors un dispositif 1 particulier permettant d'administrer des principes actifs à travers la sclère 502, et uniquement à travers la sclère 502, tel qu'un dispositif 1 proche de celui décrit dans le document FR 2 773 320, à savoir comprenant :
- une électrode active 10 annulaire de sorte à être positionnée en vis à vis de la sclère 502 ;
- un réservoir 20 formé d'une paroi latérale externe et d'une paroi latérale interne de sorte que l'électrode active 10 puisse être contenue entre elles, le fond du réservoir étant l'électrode active 10 ou une paroi transversale reliant une extrémité de la paroi latérale externe à une extrémité de la paroi latérale interne.

Selon une autre variante l'électrode active 10 n'est pas annulaire, et le réservoir 20 a une unique paroi latérale externe de sorte que l'électrode active 20 puisse être contenue dans le réservoir 10, le réservoir étant fermée à une extrémité soit par une paroi transversale formant un fond de réservoir soit par l'électrode active 10 (c'est précisément un réservoir conforme à celui représenté sur la figure 2).

En référence à la figure 3, est présentée une électrode active 10 comprenant les deux couches suivantes :
- une couche électriquement conductrice 12 agencée de sorte à recevoir un champ électrique apte à la polariser suffisamment pour électrolyser les principes actifs en solution dans le réservoir 20 (non montré ici) ; et
- une couche de protection 11, solidaire de la couche conductrice 12, cette couche de protection 11 réagissant sensiblement moins que la couche conductrice 12 lors d'une électrolyse dans la solution considérée.

Une connexion électrique 50 est prévue sur la couche conductrice 12 (par soudure par exemple) permettant ainsi de relier une liaison électrique 60 à l'électrode active 10.

Le matériau qui constitue la couche de protection 11 est particulièrement choisi pour ne pas ou très peu s'oxyder ou s'éroder lors d'une électrolyse, tout en étant suffisamment conducteur pour ne pas réduire outre mesure l'application du champ électrique fourni par la couche conductrice 12.

On pourra par exemple choisir un matériau contenant au moins en partie du carbone, tel que par exemple un polymère intrinsèquement conducteur tel qu'un polyacétylène ou un polyaniline ou un polypyrrol ou un polyphénylène ou un polythiophène ou un polymère chargé avec du noir de carbone ; ou une fibre de carbone ; ou un graphite ; ou un DLC (diamônd-like carbon) encore appelé "Carbone-diamant".

On pourra par exemple choisir un matériau semiconducteur.

La couche de protection 11 a une épaisseur pouvant être comprise entre 0,1 nanomètres et 0,5 millimètres environ.

La couche conductrice 12 peut être constituée en tout matériau qui est bon conducteur, tel un métal comme par exemple l'Argent.

La couche conductrice 12 a une épaisseur pouvant être comprise entre 0,1 nanomètres et quelques millimètres.

La couche de protection 11 recouvre avantageusement toute la couche conductrice 12.

La couche de protection 11, une fois l'électrode active 10 placée dans le réservoir, recouvre avantageusement tout le fond du réservoir.

Ce type d'électrode 10 permet d'augmenter le rendement de la iontophorèse par rapport à :
- une électrode constituée uniquement en carbone (cette dernière n'étant pas assez conductrice, n'offrant ainsi pas assez d'homogénéité de courant lorsqu'on l'alimente localement, et provoquant une administration de médicament non homogène, particulièrement dans les tissus ciblés, à savoir le pourtour immédiat et circulaire du limbe cornéo-scléral 503), puisque la couche conductrice 12 assure ici une forte densité de courant et une bonne homogénéité du courant du fait de sa forte conductivité électrique ;
- une électrode en Ag ou AgCl puisque la solution n'est pas ici nécessairement saline (comme c'est le cas avec des électrodes Ag/AgCl), ce qui implique une diminution de la compétition entre les principes actifs ionisés et les ions sodium et chlorure.

L'augmentation du rendement de iontophorèse est très bénéfique puisqu'il permet de diminuer le temps d'application. En effet, en iontophorèse oculaire, il est important de respecter des temps d'application limités typiquement à 10 minutes (préconisés dans le brevet FR 2 773 320), puisqu'un dépassement de ce temps d'application entraînerait un risque de perturber de façon significative le rôle physiologique du film lacrymal dans l'hydratation des muqueuses oculaires, au risque de provoquer une inflammation cornéenne importante. En outre, cette diminution du temps d'application diminue l'inconfort du patient.

D'autre part, la couche de protection 11 n'est pas réalisée en un matériau oxydable pour les niveaux de courant utilisés, et ne génère donc pas d'ions potentiellement toxiques (comme des particules métalliques pouvant par exemple perturber le fonctionnement de la rétine ou irriter la conjonctive).

De plus, l'électrode active 10 selon l'invention n'utilise aucun matériau noble, tel que l'or ou la platine, ce qui la rend moins coûteuse économiquement. A ce propos, le Demanderesse a mis en évidence, lors d'essais cliniques, qu'une utilisation d'or à l'électrode passive 40 provoquait néanmoins une réaction d'oxydation, alors que ce n'était pas le cas lorsque cette électrode passive 40 était en carbone.

En outre, l'électrode active 10 selon l'invention est applicable aux médicaments lipophiles, contrairement au cas d'une électrode active en Ag/AgCl pour laquelle les ions chlorure et sodium n'existent que sous forme aqueuse.

La couche de protection 11 offre ainsi une interface parfaite avec le médicament contenu dans le réservoir du dispositif pour réaliser l'électrode.

Cette électrode active 11 selon l'invention a une forme adaptée à la surface de l'oeil que l'on souhaite traitée.

Le dimensionnement et la forme de l'électrode active 10 sont ainsi agencés de sorte que celle-ci recouvre par projection au moins une partie de la cornée 501, ou au moins une partie de la cornée 501 et au moins une partie de la sclère 502, ou au moins une partie de la sclère 502.

La dernière éventualité serait préférée du fait des avantages procurés par une iontophorèse à travers la sclère 502, tel que discuté ci-dessus.

Dans une première configuration, on pourra choisir de réaliser l'électrode active 10 en enduisant un fil conducteur (représentant ici la couche conductrice 12) avec un matériau réagissant sensiblement moins que le matériau constituant le fil conducteur lors d'une électrolyse dans la solution considérée, formant ainsi la couche de protection 11 sur la couche conductrice 12.

Une telle électrode active 10 pourrait ainsi être réalisée à partir d'un fil conducteur en Argent et d'une couche de protection en Carbone.

Une architecture possible d'une telle électrode active serait un réseau de fils, chacun réalisé comme précédemment décrit (fil conducteur recouvert) dont la consistance pourrait ressembler à un tissu, et dont le coeur des fibres (ou des fils) serait alimenté en courant.

Dans une deuxième configuration, on choisira une électrode active 10 surfacique, afin de mieux répartir la densité de courant de façon homogène sur la surface à traiter.

En référence aux figures 4a à 4f sont présentés des formes particulières qui pourraient être données à une électrode surfacique, tel qu'une forme en disque ou en ellipse (figure 4a) ou une forme en portion d'anneau (figure 4b) ou en anneau (figure 4c).

En référence aux figures 4d et 4e, une liaison filaire 60 est prévue pour relier électriquement l'électrode active 10 à une source d'alimentation électrique adaptée (non représentée), la connexion électrique 50 de la liaison filaire 60 avec l'électrode active étant faite localement au niveau de la couche conductrice 12. Cette connexion 50 est ici réalisée par l'intermédiaire d'une pièce déportée 15 par rapport à l'électrode active 10 de sorte à ne pas se trouver dans le réservoir (une fois que l'électrode y est placé), une extrémité de la pièce déportée 15 étant en liaison électrique avec la couche conductrice 12 de l'électrode active 10, l'autre extrémité de la partie déportée recevant la liaison filaire 60. Ainsi, on peut déporter la connexion 50 du réservoir et s'affranchir ainsi des effets néfastes potentiels de la connexion électrique (chaleur dégagée, courants de fuite locaux, ...).

En référence à la figure 4f, la couche conductrice 12 est sous forme d'une grille ou d'un réseau.

L'électrode active 10 est optionnellement suffisamment souple pour pouvoir se déformer sous l'action de forces mécaniques de type de celles qui sont exercées lors de l'application du dispositif sur l'oeil.

L'électrode active 10 est avantageusement agencée pour avoir, en opération, une densité de courant d'environ 10mA/cm², et pour être polarisée pendant environ 10 minutes.

L'électrode active 10 peut être disposée sur le fond du réservoir 20.

L'électrode active 10 peut être formée directement sur le fond du réservoir. A cet effet, on pourra utiliser une des techniques suivantes :
- électrodéposition pour former la couche conductrice puis projection de particules pour former la couche de protection ;
- dépôts successifs d'une encre chargée en un matériau électriquement conducteur pour former la couche conductrice, puis d'une encre chargée en un matériau moins électriquement conducteur pour former la couche de protection ;
- dépôts successifs d'un film solide chargé en un matériau électriquement conducteur pour former la couche conductrice, puis d'un film solide chargé en un matériau moins électriquement conducteur pour former la couche de protection ;
- surmoulages successifs de, respectivement, polymères chargés en un matériau électriquement conducteur pour former des couches conductrices, et de polymères chargés en un matériau électriquement moins conducteur pour former des couches de protection.

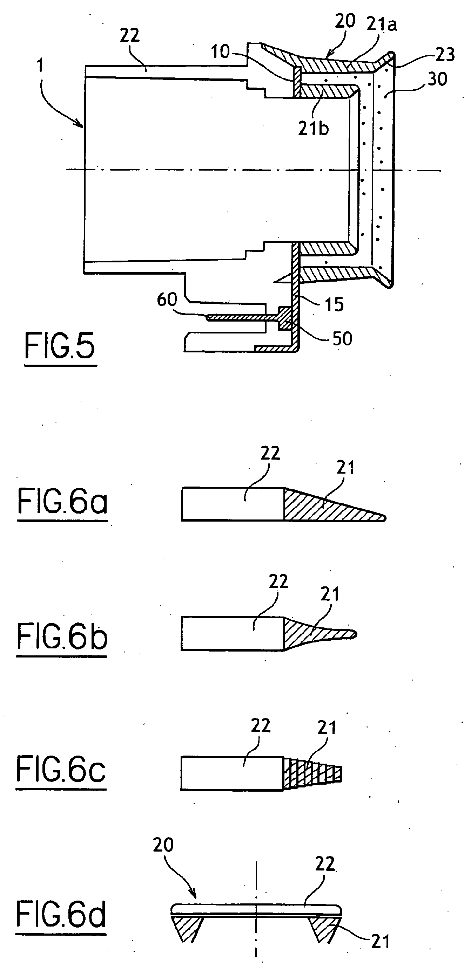

En référence à la figure 5, est représenté un dispositif de iontophorèse oculaire 1 dans lequel l'électrode active 10 présente une ouverture traversante de sorte à avoir une structure annulaire et est placée au fond du réservoir 20, lui aussi de forme annulaire.

Le dispositif 1 est avantageusement agencé de sorte que l'électrode active 10 est située autour de 4 mm de la surface oculaire lorsque le dispositif 1 est en opération, le courant de l'électrode active 10 selon l'invention n'excédant pas 10mA/cm², et le temps d'application ne dépassant pas 10min.

L'électrode active 10 comprend une partie déportée 15 permettant de déporter la connexion 50 avec la liaison filaire 60 d'alimentation électrique hors du réservoir (et donc hors de la solution contenant les principes actifs 30), tel que déjà discuté précédemment (en référence aux figures 4d et 4e).

Le réservoir 20 comprend une paroi latérale externe 21 a et une paroi latérale interne 21 b de sorte que l'électrode active puisse être contenue entre elles.

Optionnellement, l'extrémité libre de la paroi latérale interne 21 b est légèrement en retrait par rapport à l'extrémité libre de la paroi latérale externe 21a, de sorte que l'ouverture du réservoir 20 (entre ces extrémités libres) définisse ainsi une surface courbée concave épousant de façon sensiblement complémentaire la forme courbée convexe de la surface oculaire.

Une extrémité de la paroi latérale externe 21 a peut être reliée à une extrémité de la paroi latérale interne 21 b par une paroi transversale formant un fond de réservoir. L'électrode active 10 étant alors positionnée ou formée sur ce fond de réservoir.

Dans une variante, l'électrode active 10 est positionnée ou formée entre les parois latérales 21 a et 21 b du réservoir de sorte à réaliser un fond de réservoir.

Optionnellement, la paroi latérale interne 21 b du réservoir 20 présente un diamètre interne moyen dᵢ avec D<dis1,2D, D étant le diamètre d'une cornée 501.

Dans ce cas, la iontophorèse est exclusivement mise en oeuvre à travers la sclère 502.

Optionnellement, la paroi latérale externe 21 a du réservoir 20 présente un diamètre externe moyen de avec 1,4D<de<1,8D.

Selon un autre aspect de l'invention, et toujours en référence à la figure 5, les parois latérales 21 a et 21 b du réservoir 20 sont suffisamment souples pour se conformer à la surface de l'oeil.

En outre, le réservoir 20 a une partie arrière 22 renforcée ou rigide apte à résister suffisamment aux pressions exercées par les paupières.

L'électrode active 10 est ici intercalée entre ces deux parties du réservoir 20, reposant sur la partie arrière rigide 22.

Ainsi, lorsque le réservoir 20 est en position, la distance séparant la surface de l'électrode active 10 de la surface de l'oeil peut être gardée à peu près constante malgré les contraintes mécaniques exercées par les paupières.

L'anneau formé par l'électrode active 10 doit en effet, sous la pression provoquée par les paupières oculaires, maintenir sa forme, donc la surface d'application et la distance d'environ 4mm par rapport au tissus à traiter (voir ci-dessus) au risque de provoquer un court-circuit par la création de lignes de courant favorables entre l'électrode active 10 et les tissus.

Les parois latérales externes souples peuvent en outre jouer le rôle de barrière aux contaminants et au liquide lacrymal extérieurs pouvant perturber le fonctionnement du dispositif 1 (effet d'arcs). Ces parois latérales souples forment ainsi une barrière à la fuite de courant hors du réservoir 20 et/ou à l'intrusion de contaminants extérieurs dans le réservoir 20.

Les parois latérales souples 21a et 21b peuvent être formées en Silicone, ce matériau convenant très bien à un contact oculaire.

Mais sa souplesse ne permet certainement pas un maintien de la géométrie de façon précise.

C'est pourquoi il est utile de prévoir ladite partie arrière rigide ou renforcée 22, en un matériau tel que par exemple du polyméthacrylate de méthyle (encore noté PMMA), ou du Silicone de type polydiméthylsiloxane (encore noté PDMS).

Le PMMA est un matériau rigide, qui convient pour maintenir la forme de l'électrode active 10. En revanche, il ne conviendrait pas pour réaliser les parois latérales souples 21a et 21b (ce serait un matériau trop traumatique pour la délicate muqueuse oculaire).

La combinaison des deux matériaux offre ainsi une structure de dispositif tout à fait adapter à la iontophorèse oculaire.

La partie rigide 22 du réservoir 20 peut être réalisée par exemple par usinage, moulage, coulée sous vide, ou tout autre méthode de mise en oeuvre des matériaux polymères de nature rigide ou semi-rigide comme le polystyrène (encore noté PS), l'Acrylonitrile-Butadiène-Styrène (encore noté ABS), le polyéthylène (encore noté PE), le polypropylène (encore noté PP), le polyamide (encore noté PA), le polycarbonate (encore noté PC), le PMMA, le polyuréthanne (encore noté PUR)...etc.

Lors de la fabrication de cette partie, on pourra prévoir de mouler des moyens de remplissage du réservoir 20 de principes actifs 30 et/ou des moyens de circulation des principes actifs 30 dans le réservoir 20. On pourra par exemple prévoir des tubes d'amenée du principe actif 30, et éventuellement des tubes de sortie de principe actif 30.

L'électrode active 10, composée d'une couche de matériau conducteur 12 et d'une couche de protection 11, peut ensuite être déposée sur la surface de la pièce formant le fond du réservoir de médicament, en utilisant l'un des procédés cités plus haut.

Enfin, la partie souple 21 a-21 b peut être réalisée en matériau polymère, tel que par exemple un polymère élastomérique de type PUR, du Polyéther bloc Amide (encore noté PEBA), du Silicone (encore noté SI), du Styrène-éthylène-butadiène-styrène (encore noté SEBS) et peut être rapportée sur l'ensemble selon tout procédé de mise en oeuvre convenable, par exemple collage, soudage (par exemple par ultrason, ou par rotation, ou par miroir) ou surmoulage.

La partie souple 21 a-21 b du réservoir 20 peut-être également réalisée par apports successifs de matériaux de sections et de dureté progressives, respectivement, du plus épais au moins épais et du plus rigide au plus souple, de sorte à réaliser un réservoir ayant une rigidité progressivement croissante à partir de la surface à traiter (voir ci-après).

Optionnellement, des parois internes au réservoir 20 sont prévues de sorte à y délimiter des compartiments, l'électrode active 10 étant alors divisée en portions d'électrode active, chaque portion d'électrode active étant apte à être disposée dans un compartiment propre. On pourra ainsi réaliser des traitements spécifiques à partir de différents principes actifs 30, chacun répartis dans un compartiment, et administré simultanément ou en différé (dans ce dernier cas, chaque portion d'électrode a un contrôle de courant propre). Avantageusement, des moyens de remplissage et/ou de circulation de principe actif 30 sont prévus dans chaque compartiment.

Selon un aspect particulier de l'invention, les parois latérales souples 21 a et 21 b du réservoir 20 sont progressivement plus rigides lorsqu'on s'éloigne progressivement de la surface d'application du dispositif 1 en opération (c'est à dire de l'ouverture du réservoir 20).

En référence aux figures 6a à 6d, plusieurs exemples de ces parois latérales 21 à rigidité croissante sont présentés, chacune ayant une section progressivement de plus en plus grande en éloignement de l'ouverture du réservoir 20.

En référence à la figure 6a, la paroi latérale 21 forme ainsi une rampe progressive en éloignement de l'ouverture du réservoir 20.

En référence à la figure 6b, la paroi latérale 21 forme ainsi une lèvre de section de plus en plus grande en éloignement de l'ouverture du réservoir 20, et dont les côtés sont concaves.

En référence à la figure 6c, la paroi latérale 21 est ainsi formée de couches successives de sections de plus en plus grandes (en éloignement de l'ouverture du réservoir 20). Eventuellement, ces différentes couches peuvent avoir une dureté de plus en plus grande.

En référence à la figure 6d, on a ici l'exemple d'un réservoir 20 dont les parois latérales 21 ont une rigidité croissante en éloignement de l'ouverture du réservoir 20, et dont une partie rigide 22 vient fermer le réservoir 20 à une extrémité, tout en le renforçant face aux forces mécaniques des paupières oculaires.

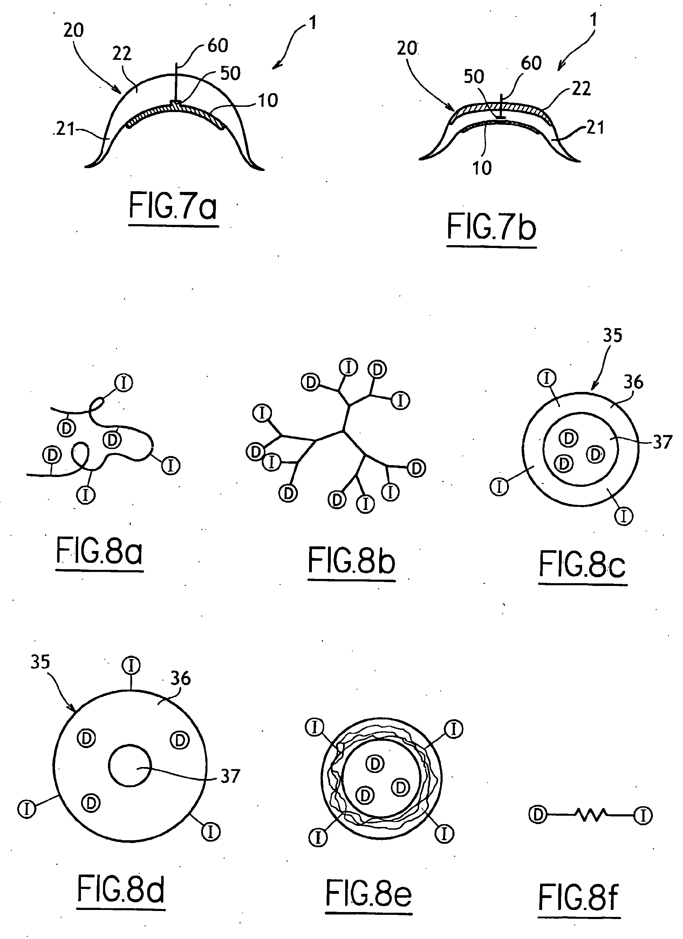

En référence à la figure 7a, des variantes de parties rigides ou renforcées 22 d'un réservoir 20 y sont présentées.

En référence à la figure 7a, le dispositif 1 comprend un réservoir 20 qui a ici une unité compacte, incluant une partie fine (souple) formant des lèvres 21 destinée à être mise en contact avec le globe oculaire 500, et une partie arrière 22 plus épaisse (rigide) destinée à contrecarrer les forces exercées par les paupières qui se ferment. La forme globale de l'électrode active 10 est courbe, avec un rayon de courbure interne proche du rayon de courbure de la cornée 501. L'électrode active 10 est disposée dans le creux du réservoir 20.

En référence à la figure 7b, le dispositif 1 est sensiblement identique à celui de la figure 7a, à l'exception que, ici, la rigidité de la partie arrière 22 est trouvée par l'apport d'un nouveau matériau plus dur venant renforcer le dispositif.

Un autre aspect de l'invention porte sur Le contenu du réservoir 20, à savoir la forme que le principe actif 30 peut prendre pour favoriser son administration dans les tissus oculaires, et particulièrement dans la rétine.

En ophtalmologie, des exemples de médicaments pouvant être concernés par ce mode d'administration sont donnés ci-après : anti-inflammatoires (dexaméthazone, méthylprednisolone hemisuccinate, betamethasone, triamcinolone...etc) ; anti-allergiques ; anti-glaucomateux ; antiangiogéniques et produits agissants sur l'endothélium néovasculaire (rétinoblastome, maladies dégénératives de la rétine liées à l'âge, rétinophaties diabétiques) ; antibiotiques ; antifongiques ; antiviraux ; neuroprotecteurs.

En outre, de nombreuses molécules sont en cours de développement pour freiner voire pour arrêter la néovascularisation observée dans les pathologies dégénératives de la rétine. Ces molécules peuvent également être transférées par iontophorèse et entrer donc dans le cadre de l'invention.

En outre, certains produits peuvent également faciliter le diagnostic des maladies à caractère prolifératif ou des conséquences du diabète. Le diagnostic actuel est effectué par l'injection systémique de fluorescéine permettant le diagnostic visuel de l'état des tissus du segment antérieur de l'oeil. Il est aussi possible d'administrer ces produits par iontophorèse.

L'électromigration occurant lors de la iontophorèse est relative au transport des charges et dépend de la valence du produit.

### +++ Dans le cas des principes actifs hydrosolubles.

Il est préférable de s'assurer de la présence d'une espèce chargée en solution, d'après la loi de dissociation.

En pratique, l'oeil est capable de supporter une gamme de pH assez large, entre 4,5 et 11,5, ceci grâce au système tampon du liquide lacrymal (qui a un pH d'environ 7,4) et au lavage de la surface oculaire par celui-ci.

Cependant, toute application d'un dispositif sur la surface oculaire aura pour effet de limiter fortement ce mécanisme.

On préférera donc en pratique pour la solution une gamme de pH comprise entre environ 6,5 et environ 8,5.

En conséquence, si la constante de dissociation acide pKa du médicament n'est pas supérieure à ces valeurs ou la constante de dissociation basique pKb n'est pas inférieure à ces valeurs, la proportion d'espèces ionisées en solution sera faible.

On choisira donc des principes actifs ayant un pKa compris entre environ 5,5 et environ 9,5.

Il est alors possible d'utiliser une forme acide ou basique du médicament à administrer ou de lier la molécule active (notée D) à un adjuvant (et à un ligand, noté I, neutralisant électriquement l'ensemble) tel qu'un polymère (voir figure 8a), un dendrimère (voir figure 8b), une nanoparticule de polymère ou une microsphère (voir figure 8e), ou un liposome (en référence à la figure 8c, le médicament est alors contenu dans le coeur aqueux 37 et non dans la paroi 36 du liposome 35), présentant des terminaisons ioniques.

En référence à la figure 8f, le médicament peut aussi être modifié, et présenter alors une structure intermédiaire entre la molécule active D et le ligandI.

+++ Dans le cas des principes actifs lipophiles, il est également possible de reformuler le médicament sous la forme d'émulsions anioniques ou cationiques (le médicament est alors dissous dans la phase huileuse de l'émulsion d'huile dans l'eau) ou sous la forme de liposomes (en référence à la figure 8d, le médicament est alors contenu dans la paroi 36 du liposome 35 et non dans le coeur aqueux 37), également chargés positivement ou négativement.

+++ Dans le cas des produits neutres, on ne bénéficie que du mécanisme d'électroosmose. Dans ce cas, il est préférable d'utiliser une forme ionisée pour la molécule active, comme le Dexaméthasone phosphate, plutôt que le Dexaméthasone qui est neutre en solution.

Enfin, la concentration du produit devra être la plus élevée possible, le seul facteur limitant étant la solubilité et la tolérance oculaire, tel que le poids moléculaire maximal des particules à administrer autorisé par la membrane sclérique, ou l'irritation des muqueuses engendrée par le contact du produit.

## Revendications

1. Dispositif d'iontophorèse oculaire (1), permettant d'administrer un médicament dans un oeil et comprenant :
un réservoir (20), le réservoir ayant une partie avant souple (21) et une partie arrière (22), la partie avant souple formant des lèvres (21) à mettre en contact avec l'oeil ;
une électrode active (10) disposée dans un creux du réservoir, **caractérisé en ce que** l'électrode active (10) est courbe et dans lequel l'électrode active présente un rayon interne de courbure proche du rayon de courbure de la cornée de l'oeil ; et
un médicament (30) stocké dans le réservoir.

2. Dispositif d'iontophorèse oculaire selon la revendication 1, dans lequel l'électrode active (10) comprend :
une couche conductrice d'électricité (12); et
une couche de protection (11) couplée à la couche conductrice d'électricité (12), la couche de protection réagissant considérablement moins que la couche conductrice pendant l'administration du médicament par iontophorèse.

3. Dispositif d'iontophorèse oculaire selon la revendication 2, dans lequel le médicament (30) est stocké entre la couche de protection (11) et la partie avant (21) du réservoir.

4. Dispositif d'iontophorèse oculaire selon la revendication 2, dans lequel la couche de protection (11) comprend :
un matériau qui ne s'érode pas pendant l'administration du médicament par iontophorèse.

5. Dispositif d'iontophorèse oculaire selon la revendication 4, dans lequel le matériau comprend:
au moins un élément parmi une polyacétyline, une polyaniline, un polypyrrole, un polyphenylène, un polythiophène, un polymère rempli de noir de carbone, une fibre de carbone, un graphite et un dépôt CDA.

6. Dispositif d'iontophorèse oculaire selon la revendication 2, dans lequel la couche de protection (11) recouvre sensiblement la couche conductrice d'électricité (12).

7. Dispositif d'iontophorèse oculaire selon la revendication 1, dans lequel l'électrode active (10) est positionnée ou formée sur la partie arrière (22) du réservoir (20).

8. Dispositif d'iontophorèse oculaire selon la revendication 1, dans lequel le médicament (30) se trouve dans un gel ou une solution.

9. Dispositif d'iontophorèse oculaire selon la revendication 1, comprenant en outre un élément de renfort couplé à un côté opposé de la partie arrière (22) du réservoir (20).

## Patentansprüche

1. Okulare Iontophoresevorrichtung (1), die das Verabreichen eines Arzneimittels in ein Auge ermöglicht und Folgendes umfasst:
ein Reservoir (20), wobei das Reservoir einen weichen vorderen Teil (21) und einen hinteren Teil (22) aufweist, wobei der weiche vordere Teil Lippen (21) zum Inberührungkommen mit dem Auge bildet;
eine aktive Elektrode (10), die in einem Hohlraum des Reservoirs angeordnet ist, **dadurch gekennzeichnet, dass** die aktive Elektrode (10) gekrümmt ist, und wobei die aktive Elektrode einen inneren Krümmungsradius hat, der dem Krümmungsradius der Kornea des Auges nahe kommt; und
ein Arzneimittel (30), das in dem Reservoir aufbewahrt ist.

2. Okulare Iontophoresevorrichtung nach Anspruch 1, wobei die aktive Elektrode (10) Folgendes umfasst:
eine elektrisch leitfähige Schicht (12) und
eine Schutzschicht (11), die mit der elektrisch leitfähigen Schicht (12) verbunden ist, wobei die Schutzschicht während der Verabreichung des Arzneimittels durch Iontophorese wesentlich weniger als die leitfähige Schicht reagiert.

3. Okulare Iontophoresevorrichtung nach Anspruch 2, wobei das Arzneimittel (30) zwischen der Schutzschicht (11) und dem vorderen Teil (21) des Reservoirs aufbewahrt wird.

4. Okulare Iontophoresevorrichtung nach Anspruch 2, wobei die Schutzschicht (11) Folgendes umfasst:
ein Material, das während der Verabreichung des Arzneimittels durch Iontophorese nicht erodiert.

5. Okulare Iontophoresevorrichtung nach Anspruch 4, wobei das Material Folgendes umfasst:
mindestens ein Element von einem Polyacetylen, einem Polyanilin, einem Polypyrrol, einem Polyphenylen, einem Polythiophen, einem mit Ruß gefüllten Polymer, einer Kohlefaser, einem Graphit und einer DLC-Ablagerung.

6. Okulare Iontophoresevorrichtung nach Anspruch 2, wobei die Schutzschicht (11) die elektrisch leitfähige Schicht (12) im Wesentlichen abdeckt.

7. Okulare Iontophoresevorrichtung nach Anspruch 1, wobei die aktive Elektrode (10) auf dem hinteren Teil (22) des Reservoirs (20) positioniert oder ausgebildet ist.

8. Okulare Iontophoresevorrichtung nach Anspruch 1, wobei das Arzneimittel (30) sich in einem Gel oder einer Lösung befindet.

9. Okulare Iontophoresevorrichtung nach Anspruch 1, die außerdem ein Verstärkungselement umfasst, das mit einer Seite verbunden ist, die entgegengesetzt zu dem hinteren Teil (22) des Reservoirs (20) ist.

## Claims

1. Ocular iontophoresis device (1) allowing a drug to be administered in an eye and comprising:
a reservoir (20), the reservoir having a front flexible part (21) and a back part (22), the front flexible part forming lips (21) to be placed in contact with the eye;
an active electrode (10) disposed in a recess in the reservoir,
**characterised in that** the active electrode (10) is curved and in which the active electrode presents an internal curvature radius close to the curvature radius of the cornea of the eye; and
a drug (30) stored in the reservoir.

2. Ocular iontophoresis device according to claim 1, in which the active electrode (10) comprises:
an electricity-conducting layer (12); and
a protective layer (11) coupled to the electricity-conducting layer (12), the protective layer reacting considerably less than the conductive layer during administration of the drug by iontophoresis.

3. Ocular iontophoresis device according to claim 2, in which the drug (30) is stored between the protective layer (11) and the front part (21) of the reservoir.

4. Ocular iontophoresis device according to claim 2, in which the protective layer (11) comprises:
a material that does not erode during administration of the drug by iontophoresis.

5. Ocular iontophoresis device according to claim 4, in which the material comprises:
at least one element from a polyacetylene, a polyaniline, a polypyrrole, a polyphenylene, a polythiophene, a polymer filled with carbon black, a carbon fibre, a graphite and a DLC deposit.

6. Ocular iontophoresis device according to claim 2, in which the protective layer (11) substantially covers the electricity-conducting layer (12).

7. Ocular iontophoresis device according to claim 1, in which the active electrode (10) is positioned or formed on the back part (22) of the reservoir (20).

8. Ocular iontophoresis device according to claim 1, in which the drug (30) is in the form of a gel or solution.

9. Ocular iontophoresis device according to claim 1, furthermore comprising a reinforcing element coupled to an opposite side of the back part (22) of the reservoir (20).
